(19) **Europäisches Patentamt / European Patent Office / Office européen des brevets**

(11) **EP 3 419 695 B1**

(12) # EUROPEAN PATENT SPECIFICATION

(45) Date of publication and mention of the grant of the patent:
**07.04.2021 Bulletin 2021/14**

(21) Application number: **17757310.2**

(22) Date of filing: **24.02.2017**

(51) Int Cl.:
*A61M 5/142* (2006.01)     *A61K 9/00* (2006.01)

(86) International application number:
**PCT/US2017/019373**

(87) International publication number:
**WO 2017/147436 (31.08.2017 Gazette 2017/35)**

(54) **COMPACT PLATFORM FOR ACCURATE DRUG DELIVERY**

KOMPAKTE PLATTFORM ZUR GENAUEN ARZNEIMITTELABGABE

PLATE-FORME COMPACTE POUR ADMINISTRATION PRÉCISE DE MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2016 US 201662300542 P**

(43) Date of publication of application:
**02.01.2019 Bulletin 2019/01**

(73) Proprietor: **Progenity, Inc.**
**San Diego CA 92122 (US)**

(72) Inventors:
• **IORDANOV, Ventzeslav, Petrov**
  **5551 TZ Valkenswaard (NL)**
• **ILIEV, Blagoy, P.**
  **2625 CE Delft (NL)**
• **KERKHOF, Klaas**
  **5672 RH Nuenen (NL)**
• **SHIMIZU, Jeffrey, A.**
  **Charlotte, NC 28278 (US)**

• **FIENIEG, Robert**
  **Veldhoven (NL)**
• **BRINKERT, Jacob**
  **7433 MP Schalkhaar (NL)**
• **TONNIE TE VRUCHTE - HAJ MODIRI, RONALD, F.A.**
  **Rosmalen (NL)**

(74) Representative: **Mewburn Ellis LLP**
**Aurora Building**
**Counterslip**
**Bristol BS1 6BX (GB)**

(56) References cited:
| | |
|---|---|
| US-A- 4 265 241 | US-A- 5 049 141 |
| US-A- 5 607 418 | US-A- 5 704 520 |
| US-A1- 2003 216 714 | US-A1- 2003 216 714 |
| US-A1- 2006 013 710 | US-A1- 2007 299 408 |
| US-A1- 2011 160 668 | US-A1- 2014 194 851 |

**Description**

**CROSS-REFERENCE TO RELATED APPLICATIONS**

The present application is an International Application claiming priority to U.S. Provisional Patent Application No. 62/300,542 filed February 26, 2016.

**BACKGROUND**

Administering accurate dosages of medicine or other therapeutic substances is often a key element to effective treatment of various maladies. Many conventional drug delivery devices provide for complete, one-time delivery of a drug payload contained in a reservoir. However, many applications would benefit from distributing the payload as a series of smaller, controlled doses. While some conventional delivery devices aim for metered drug delivery, they tend to be overly complex and/or bulky to be practical.

Accordingly, there is a need in the art for effective drug delivery devices capable of providing accurately-controlled doses of a therapeutic substance contained in a reservoir. This disclosure is directed to overcoming one or more problems set forth above and/or other problems of the prior art.

**BRIEF SUMMARY**

This application describes improved drug delivery devices. In some embodiments, a drug delivery device herein may be embodied as a self-contained device for placement in a capsule configured for insertion into a patient. For example, the capsule may be swallowable or otherwise inserted into a bodily cavity. The drug delivery device may include a reservoir containing a substance to be dispensed and a compressible dispensing chamber in fluid communication with the reservoir. The compressible dispensing chamber defines a dispensing volume and includes a dispensing chamber inlet in fluid communication with the reservoir and a dispensing chamber outlet in fluid communication with the environment. An actuator is configured to compress the compressible dispensing chamber to decrease the dispensing volume and expel the substance in the dispensing chamber through the dispensing chamber outlet. Upon deactivating the actuator, the compressible dispensing chamber returns to its original, non-compressed configuration, and refills with the substance from the reservoir.

Further areas of applicability of the present disclosure will become apparent from the detailed description provided hereinafter. It should be understood that the detailed description and specific examples, while indicating the preferred embodiment of the invention, are intended for purposes of illustration only and are not intended to limit the scope of the invention. US 5 049 141 A relates to a pump comprising separate valves with a diaphragm held in position by a spacer plate. Grooves allow fluid communication between the inlet and outlet.

The invention provides a pump according to claim 1 and a fluid delivery device according to claim 10.

**BRIEF DESCRIPTION OF THE DRAWINGS**

The present invention will become more fully understood from the detailed description and the accompanying drawings, wherein:

FIG. 1 is a schematic illustration of a delivery device according to an example implementation of this disclosure;
FIG. 2 is an exploded perspective view of a delivery according to another example implementation of this disclosure;
FIG. 3 includes electrical schematics of driving circuits for delivery devices such as those illustrated in FIGS. 1 and 2, according to example implementations of this disclosure; and
FIG. 4 is a flow chart illustrating a method of driving a delivery device according to implementations of the present disclosure.

**DETAILED DESCRIPTION**

The following description of the preferred embodiments is merely exemplary in nature and is in no way intended to limit the disclosure, its application, or uses.

In some instances, drug delivery devices are known that include a driving mechanism used to drive a medicinal substance from a reservoir. Drug delivery devices of this type may be extracorporeal or intra-corporeal. Extracorporeal example devices may include a needle in fluid communication with the reservoir and the driving mechanism is arranged to drive the medicinal substance through the needle and into the patient. Intra-corporeal example devices may include implantable devices. Alternatively, the intra-corporeal devices may be adapted to dispense the medicinal substance in a bodily cavity. For example, the device may be embodied as an electronic capsule, and the capsule may be swallowed or otherwise inserted into a bodily cavity.

This disclosure relates generally to fluid delivery systems, and systems according to this disclosure may be incorporated into either extracorporeal or intra-corporeal devices. Although certain embodiments and benefits will be described, other implementations, modifications, and/or benefits will be appreciated those having ordinary skill in the art, with the benefit of this disclosure. Although the devices described herein may be particularly well-suited for dispensing certain types of substances, such as liquid medications, this disclosure is not limited to delivery of any specific substance or class of substances.

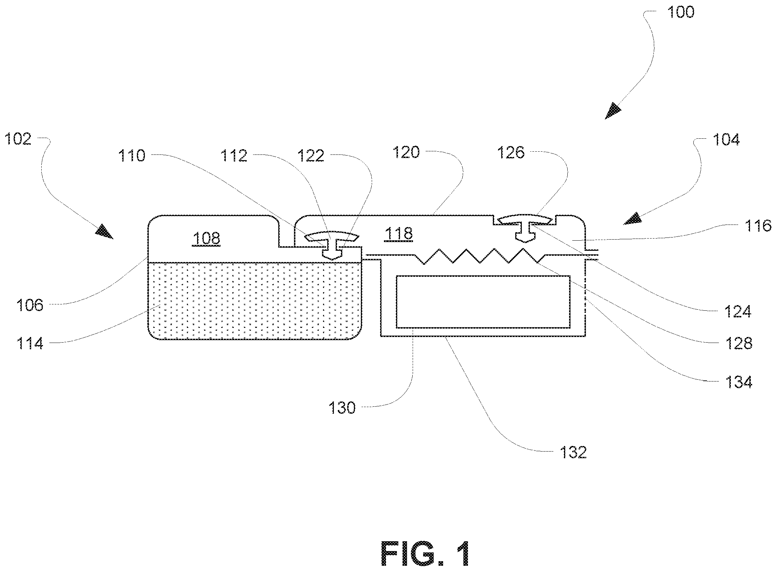

FIG. 1 is a schematic representation of a drug delivery device or pump 100 according to examples of this disclosure. The device 100 generally includes a reservoir 102 and a dispenser 104. The reservoir contains a substance 114 to be dispensed by the device 100. The dispenser 104 is disposed in fluid communication with the reservoir 102 such that contents of the reservoir may pass to the dispenser before being expelled to the environment.

The reservoir 102 includes a reservoir sidewall 106 generally defining a reservoir volume 108. A reservoir outlet 110 is formed as a hole or other opening through the reservoir sidewall 106. In some implementations, the outlet 110 may be used to fill the reservoir prior to use, although in alternative embodiments one or more additional openings may be provided through the sidewall 106 for filling purposes. A reservoir valve 112 also is provided in the reservoir outlet 110, to control flow of the substance 114 contained in the reservoir volume 108.

The reservoir sidewall 106 may be a rigid sidewall, such that the reservoir volume 108 is a fixed volume. For example, the sidewall 106 may be formed of a rigid polymer, a metal, or the like. In preferred embodiments, the sidewall 106 is made from a material that will not react with or otherwise effect or be effected by the substance 114. The material making up the reservoir sidewall 106 also may be chosen to be non-toxic, for example, for intra-corporeal applications. In other embodiments, the sidewall 106 may exhibit some flexibility.

The dispenser 108 includes a compressible dispensing chamber 116 defining a dispensing volume 118. The dispensing volume 118 is smaller than the reservoir volume 108. In the illustrated embodiment, the dispensing chamber 116 includes a sidewall 120 that at least partially defines the dispensing volume 118. A dispensing chamber inlet 122, formed as a first hole through the sidewall 120, is in fluid communication with the reservoir outlet 110. In the schematic of FIG. 1, a portion of the reservoir sidewall 106 and a portion of the dispensing chamber sidewall 120 make up a common wall between the reservoir 102 and the dispenser 104. A single hole formed through this common wall makes up both the reservoir outlet 110 and the dispensing chamber inlet 122. In other embodiments, the reservoir 102 and the dispenser 104 may be separate, and thus the reservoir outlet 110 and the dispensing chamber inlet 122 may be separate holes formed through the respective reservoir 102 and dispenser 104. Moreover, a conduit or other fluid connection may be provided between the reservoir outlet 110 and the dispensing chamber inlet 122, when those features are separated.

The dispensing chamber 116 also includes a dispensing chamber outlet 124 formed as a second hole through the sidewall 120. The dispensing chamber outlet 124 is spaced along the sidewall 120 from the dispensing chamber inlet 122. A dispensing chamber outlet valve 126 is disposed in the dispensing chamber outlet 124. In embodiments of this disclosure, the dispensing chamber outlet valve 126 is a one-way valve that opens when pressure inside the dispensing chamber 116 exceeds a pressure outside the dispensing chamber 116. However, when the pressure inside the dispensing chamber 116 is less than a pressure outside the dispensing chamber, the dispensing outlet valve 126 closes the dispensing chamber outlet 124, thereby prohibiting the flow of material into or out of the dispensing chamber, through the dispensing chamber outlet 124.

At least a portion of the dispensing volume 118 is defined by a flexible portion, which may be a flexible membrane 128. The flexible membrane is movable relative to the sidewall 120 of the dispensing chamber 116 to increase and decrease the dispensing volume 118. An actuator 130 is disposed on a side of the flexible membrane 128 opposite the dispensing volume 118 and positioned to act on the flexible membrane 128. More specifically, the actuator 130 is disposed to apply a selective pressure to the flexible membrane 128 to increase the pressure in the dispensing volume 118 and compress the dispensing volume 118 and compress the dispensing volume 118.

As illustrated in FIG. 1, the actuator 130 may be disposed in the housing 132. The housing 132 may be secured or otherwise fixed relative to the dispensing chamber 116. For example, a portion of the housing 132 and a portion of the sidewall 120 may form outer surfaces of the dispenser 104. In other embodiments, a single housing may contain the dispensing volume, the flexible membrane 128 and the actuator 130. Regardless of the arrangement, it may be preferred that the dispenser 104 includes a flexible membrane 128 separating the actuator 130 from the dispensing volume 118, and that the actuator is disposed to contact and displace the flexible membrane 128 in a manner that compresses the dispensing volume.

As illustrated in FIG. 1, the housing 132 also includes an exhaust 134. The exhaust may be formed as one or a plurality of apertures or holes formed through a sidewall of the housing 132. In some preferred embodiments, the exhaust 134 may comprise at least a portion of the sidewall of the housing 132 that is made of a porous material that allows for passage of some contents in the housing 132 to outside the housing 132. For example, some polymers are known through which vapor or gaseous materials may migrate, and some or all of the housing 132 may be formed from such a polymer. In some embodiments, the dissipation film may be made from silicone, for example. Microporous materials also are known, and could form a portion of the exhaust 134.

In some embodiments, the exhaust 134 may be provided to allow excess pressure in the housing 132 to be relieved. For example, the actuator 130 may be a gas generating cell, such as a hydrogen generating cell, and excess hydrogen in the housing may be relieved through the exhaust 134. When a gas generating cell is used, for example, the exhaust 134 may comprise a vapor permeable polymer chosen to dissipate gas therethrough at a rate slower than the rate which gas is produced. In other embodiments, the actuator may be an electro mechanical actuator, such as a servo motor. Such an actuator may give off volatile organic compounds (VOCs), and it may be desirable to dissipate those VOCs through the exhaust 134.

In use, the delivery device 100 may be provided such that the reservoir 102 and the dispensing volume 118 are prefilled with the substance 114. The delivery device 100 preferably is formed as a part of a swallowable or insertable apparatus. For example, the delivery device 100 may comprise a swallowable capsule sized and shaped for swallowing by a patient. Alternatively, the device 100 may comprise an implant or a form for insertion. In some embodiments, the device 100 may be included in a larger device, or additional features may be provided for different functionality. By way of nonlimiting example, the device 100 may include structure that allows it to be retained in the bodily cavity once inserted. For instance, the device 100 may be incorporated into a ring or other form factor designed for insertion into and retention in a female reproductive system.

When in the body, i.e., after swallowing, inserting, or implanting, the device 100 commences delivering the substance 114 by controlling the actuator to generate sufficient pressure on the flexible membrane 128 to displace the flexible membrane in a direction that increases the pressure in, and reduces the volume of, the dispensing volume 118. For example, when the actuator is a gas generator, such as a hydrogen generating cell, the amount of gas in the housing accumulates over a certain period of time, thereby increasing pressure applied to the flexible membrane 128. When a mechanical or electromechanical actuator is used, a piston or similar member of the actuator may press against the flexible membrane 128.

Through increased pressure against the flexible membrane 128, pressure in the dispensing volume 118 continues to grow until it reaches a level sufficient to exceed a force closing the dispensing chamber outlet valve 126. Once this force is exceeded, the contents of the dispensing volume 118 escape the device through the dispensing chamber outlet 124. The force closing the dispensing chamber outlet 126 (and thus overcome by the action of the actuator to expel the substance) may be the result of a pressure applied by the environment of the device 100. For instance, a pressure may be applied by the surroundings of the device 100 in the gastrointestinal tract of the patient. In other embodiments, a spring or other biasing member may act on the dispensing chamber outlet valve 126 and the valve 126 is open only when the pressure inside the dispensing volume 118 is sufficient to exceed the force of the spring or other biasing member.

With the contents of the dispensing volume 118 expelled, a force applied to the flexible membrane 128 is removed, e.g., by halting gas generation or retracting a piston. The flexible membrane therefore returns to its original position and the dispensing volume 118 returns to its original size. For instance, the flexible membrane may be naturally biased to return to its original position, e.g., by being formed of an elastic member that will return to its non-deformed shape and state. Moreover, the dispensing chamber outlet valve 126 returns to its closed position. Also at this time, any residual generated gas in the housing 132 may dissipate through the exhaust 134.

As the dispensing volume 118 returns to its non-compressed state, a pressure differential results between the reservoir volume 108 and the empty dispensing volume 118. This pressure differential is sufficiently large to open the reservoir valve 112, thereby allowing flow of liquid contained in the reservoir 102 into the dispensing volume 118. As the dispensing volume 118 fills the pressure differential between the reservoir volume 108 and the dispensing volume 118 reduces, and the reservoir valve 112 closes. In this manner, the device 100 is restored to a position at which the dispensing chamber volume is filled with a predetermined amount of substance for administration according to the method just described.

As will be appreciated by those having ordinary skill in the art, with the benefit of this disclosure, the device 100 allows for accurate and controlled drug delivery because the amount of drug to be administered is defined by the size of the dispensing volume 118, not by the force applied by the actuator, or some other condition. Moreover, by sizing the reservoir volume 108 several times larger than the dispensing volume 118, the device can accurately dispense several doses of the same substance.

Modifications and/or additions to the device 100 also are contemplated by the inventors. For example, although not illustrated, an actuator or biasing member may be provided to act on the reservoir 102. Such an actuator or biasing member may maintain a minimum pressure in the reservoir, e.g., to assist in transfer of substance 114 from the reservoir 102 into the dispensing volume 118. The fluid delivery device 100 also may include a controller and a power source for driving and powering the actuator.

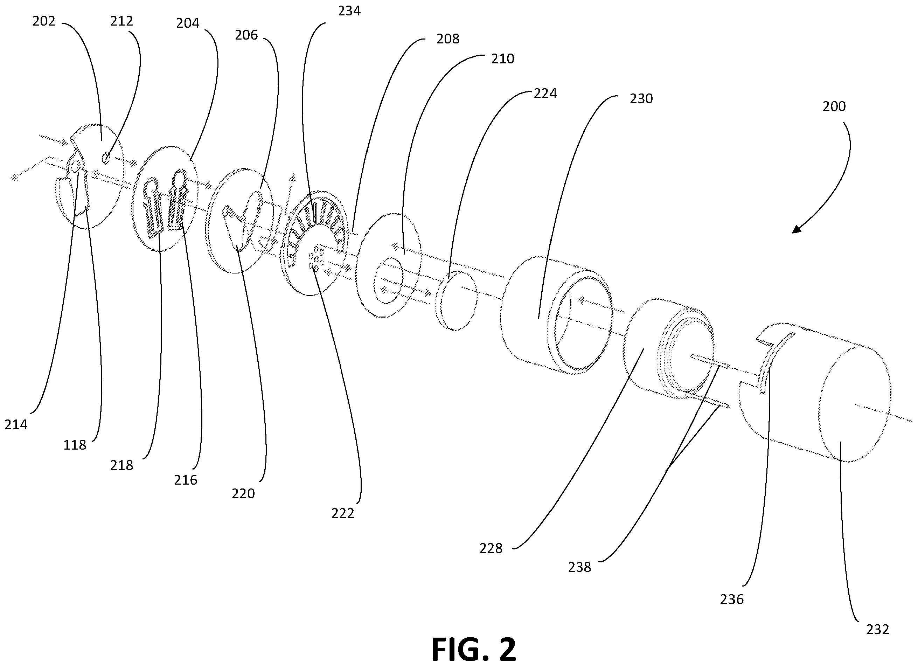

FIG. 2 illustrates another embodiment of a fluid delivery device 200 according to another embodiment of this disclosure. FIG. 2 depicts an exploded view of components forming the device 200, which may be a commercial or practical embodiment of the dispenser 104 of the device 100 shown schematically in FIG. 1. The device 200 generally includes a plurality of plates or circular disks that when assembled, provide for controlled delivery of some predetermined amount of a substance. Moreover, the device 200 preferably is in fluid communication with a reservoir (not shown in FIG. 2) that provides a supply of a substance to be dispensed. In some embodiments, the device 200 cooperates with the reservoir to provide controlled doses of the substance contained in the reservoir.

In more detail, the device 200 illustrated in FIG. 2 includes an inlet/outlet disk 202, a valve disk 204, a channel disk 206, a dissipation disk 208, and a dissipation film 210. These and other features of the device 200 will be described in more detail below.

The inlet/outlet disk 202 is a generally planar member and includes two holes formed through the disk. The first hole is an inlet 212 through which the substance to be dispensed enters the device 200. For example, the inlet 212 is in fluid communication with the reservoir (not shown) containing a substance to be dispensed by the device 200. The inlet 212 may function similarly to the dispensing chamber inlet 122 of the device illustrated in FIG. 1. The second hole is an outlet 214 through which a controlled amount of substance contained in the device 200 is expelled into the environment. The outlet 214 may function similarly to the dispensing chamber outlet 124 of the device 100, described above.

The valve disk 204 is disposed adjacent to the inlet/outlet disk 202. The valve disk 204 includes an inlet valve 216 and an outlet valve 218. The inlet valve 216 is aligned with the inlet 212 of the inlet/outlet disk 202. In some embodiments, the inlet valve 216 is a one-way valve that selectively allows a substance at the inlet 206 to enter the device 200, but prohibits flow in the in the opposite direction. More specifically, the inlet valve 216 substantially prohibits the passage of a substance out of the device 200 via the inlet 212. The outlet valve 218 is arranged to cooperate with the outlet 214. The outlet valve 218 preferably is formed as a one-way valve that allows for passage of substance contained in the device 200 out of the device 200 via the outlet 214. However, the outlet valve 218 substantially prohibits entry of any material into the device 200 via the outlet 214. The inlet valve 216 and the outlet valve 218 may function similarly to the reservoir valve 112 and the dispensing chamber outlet valve 126, respectively, of the device 100, discussed above.

The channel disk 206 is disposed on a side of the valve disk 204 opposite the inlet/outlet disk 202. The channel disk 206 has a channel 220 formed therein. The channel 220 may be a cavity or void formed in a side of the channel disk 206. A volume is defined by the channel 220 and a flexible membrane proximate the channel (described below). This volume functions similarly to the dispensing chamber volume 118 of the device 100 described previously. More specifically, the flexure of the flexible member controls the amount of substance to be dispensed by the device 200. The channel 220 is in fluid communication with both the inlet 212 and the outlet 214.

The dissipation disk 208 is formed on a side of the channel disk 206 opposite the valve disk 204. The dissipation disk includes one or more apertures 222 generally aligned with the channel 220 of the channel disk 206. The substance contained in the channel 220 may pass through the apertures 222 of the dissipation disk 208.

The dissipation film 210 is disposed on a side of the dissipation disk 208 opposite the channel disk 206. Among other features, the dissipation film 200 contains a flexible portion providing a flexible membrane 224 aligned with and covering the apertures 222 of the dissipation disk 208. The dissipation film 210 preferably is formed of a gas and/or vapor permeable material providing a controlled rate of gas/vapor permeation. In contrast, the flexible membrane 224 may be formed of a material that is not gas and/or vapor permeable in some embodiments. The flexible membrane 224 cooperates with the channel 220 to form a compressible dispensing volume.

An actuator 228 is disposed on a side of the dissipation film 210 and flexible membrane 224 opposite the dissipation disk 208. As also illustrated in FIG. 2, the device 200 may include one or more housing components 230, 232 that function to contain, align, and/or otherwise support the various components of the device 200.

The device 200 is generally assembled to provide a compact fluid delivery device 200 in which each of the discs described above and additional components are arranged adjacent to each other in the housings 230, 232. The device 200 also is provided in fluid communication with a reservoir or other supply of a substance to be delivered via the device 200. As noted above, the reservoir or other supply preferably is in fluid communication with the inlet 212 of the inlet/outlet disk 202.

The device 200 may be disposed in a swallowable capsule, for example, or some other insertable or implantable device. In other embodiments, the device 200 may be intended for use outside of a body, and thus could be incorporated into other form factors. When prepared for operation, the device 200 may be provided with an initial supply of a substance to be dispensed contained in the channel 220 of the channel disk 206. With the channel 220 filled with the substance, a pressure is applied on the flexible membrane 224 through the apertures 222 formed in the dissipation disk 208 to deflect the flexible membrane 224 toward the actuator 228. For example, when it is determined that the substance contained in the channel 220 is to be dispensed, the actuator is energized to impart a force on the flexible membrane in a direction toward the channel 220. As in embodiments described above, the actuator 228 may be a gas generating device, such as a hydrogen generating cell, or the actuator may be an electromechanical actuator, by way of non-limiting example.

As pressure accumulates on the actuator side of the flexible membrane 224, the flexible membrane 224 deflects, reducing the volume defined by the channel 220, the apertures 222, and a void formed by the deflected flexible membrane 224. As will be appreciated, the deflection of the flexible membrane 224 is restricted by the dissipation disk 208. The reduction of the volume containing the substance to be dispensed causes the substance to be forced out of channel 220 via the outlet 214, through the outlet valve 218. At this time, the actuator is caused to stop applying a pressure to the flexible membrane 224, for example by stopping gas generation, or reversing a piston.

Gas building on the actuator side of 224 is then dissipated through film 210, channels 234, and out through housing aperture 236. Flexible membrane 224 then returns to original position due to the reduction of pressure on the actuator side, or by mechanical retraction in the case of a mechanical actuator. As the flexible membrane 224 returns to its original position, a pressure differential exists between the channel 220 and the reservoir. Because of this pressure differential, liquid flows from the reservoir through the inlet 212 past the inlet valve 216 to refill the channel, thereby preparing the device 200 to deliver another dosage.

As noted above, in some implementations, the actuator may be a gas generating cell, such as a hydrogen generating cell. In these implementations, the pressure on the flexible membrane 224 that causes the membrane 224 to deflect in a direction toward the dissipation disk 208 is provided by an accumulation of the generated gas in a volume defined by one or more of the housing 230, 232 and/or the dissipation film 210. The dissipation film 210 may be chosen to dissipate the generated gas at a rate slower than the rate at which the gas is generated by the actuator 228. However, when the actuator stops generating the gas, residual gas will dissipate through the dissipation film 210 to exit the housings 230, 232. As also illustrated in FIG. 2, the dissipation disk 208 may include a series of channels 234 or other features that allow gas dissipated through the dissipation film 210 to exit the device 200 into the atmosphere. The illustrated channels 234 generally allow gas to exit through an edge of the dissipation disk 208 and the housing 232 includes a slot 236 or other opening through which gases exiting the channels 234 can further exit the device 200.

The actuator 228 may be connected to a controller (not shown) and/or power source (not shown) by one or more leads 238. The controller may be used to selectively energize and de-energize the actuator 228 to control dosageing of a substance using the device 200. The controller may be a microcontroller or other suitable controller. The controller and/or the power source may be integrated into the device, e.g., by being disposed in the housing, or may be disposed outside of the housing. When outside the housing, leads or other wired or wireless connections may be used to supply the necessary power and/or instructions to drive the actuator. In other implementations, the actuator may be a gas cell that incorporates a dedicated power source. For example, the gas cell may be shorted through a controlled resistance to start gas generation. The rate of gas generation may be controlled by the resistance, for example.

Although the device 200 is illustrated as containing a plurality of generally planar disks, other constructions will be appreciated by those having ordinary skill in the art, with the benefit of this disclosure. For example, each of the components may have a square, rectangular, or other shape. Other modifications also are contemplated. For example, when a non-gas generating actuator is used, the dissipation film 210 and the dissipation channels 234 may not be necessary. When a gas generator is used, excess gas may be dissipated other than through the dissipation film 210. For example, at least a portion of the housing 230, 232 may be formed of a gas permeable material and dissipation may occur directly through the housing. In these embodiments, the channels 234 may not be required, and the dissipation film 210 could be any material.

Moreover, in embodiments where the dissipation channels 234 are not required, the dissipation disk 208 may not be included at all. For example, the flexible membrane could be disposed directly adjacent the channel 222 with the membrane 224 completely circumscribing the channel 220.

In still other contemplated modifications, the described components of the device 200 may be provided on more or fewer disks. For example, the inlet valve 216 or the outlet valve 218 may be formed on separate disks, or one or both could be incorporated into the inlet/outlet disk 202.

As will be appreciated from the foregoing, the devices 100, 200 may provide fluid dispensing devices that achieve repeatable, controlled dosing. In the device 200, many components are formed as planar elements. These planar elements may be made, for example, from stainless steel sheets that are patterned and etched using conventional photolithographic techniques. This may allow for accurate and reproducible parts to be made in high-volume and at low-cost. Moreover, the dispensing volume of the device 200 may be readily altered by changing only the shape of the flexible membrane 224. Moreover, the flexible membrane may be designed such that in the absence of pressure by the actuator, the device 100 assumes a normal state with a cavity that defines the volume to be dispensed. This natural state also provides the restorative force to fill the dispensing volume after gas dissipation. When a gas generating actuator is used, the rate of gas generation and dissipation controls the minimum cycle time for the device 200. As will be appreciated, the rate of dissipation may be controlled by changing the thickness and/or material of the dissipation film.

In some embodiments, the gas cell is the preferred actuator due to its small size, cost, commercial availability, and/or simple electrical drive. Typically, such gas cells are driven in a simple load short arrangement. Although this creates, after time, a stable gas rate production, the rate of gas produced initially is poorly controlled. Because of this poorly controlled initial rate, gas generators are conventionally poorly suited for accurate delivery in a pulsed mode. However, by employing the device 100, 200 described above, the shortcomings of gas generators may be overcome.

To further overcome the deficiencies of conventional gas drives, and improved electrical drive method may also be used in this disclosure. For underlying principles one may make use of the Faraday's law of electrophoresis:

$$m = \left(\frac{Q}{F}\right)\left(\frac{M}{z}\right), \qquad (1)$$

where

$m$ is the mass of the substance liberated at an electrode in grams,
$Q$ is the total electric charge passed through the substance,
$F$ = 96485 C mol-1 is the Faraday constant,
$M$ is the molar mass of the substance, and
$z$ is the valency number of ions of the substance (electrons transferred per ion).

Since $M, F,$ and $z$ are constants, $m$ will be directly proportional to $Q$, that is, the produced amount of gas is directly proportional to the amount of charge driven through the cell. Based on the last the cell can then be driven accurately in so called "charge mode," integrating the current through the cell over a set period of time:

$$Q = \int_0^t I(\tau)d\tau = \int_0^t \frac{V(\tau)}{R}d\tau, \qquad (2)$$

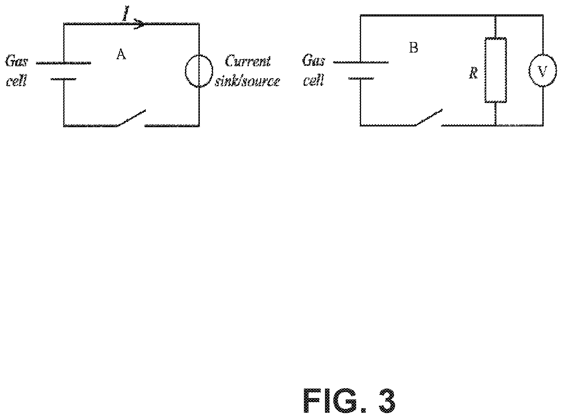

which can be based as provided above on a constant current $I$ or a fixed resistive load $R$. With taking this into account one may rely on one of the circuit bases illustrated in FIG. 3. In FIG. 3, a gas cell is driven by a A) constant current $I$ or B) through a fixed load $R$.

In some embodiments, the gas cell is driven in a start/stop pulsatile fashion. Particularly at the start of the drive the resistance of the cell changes rapidly. In order to come to the desired fixed charge application, voltage is measured periodically and with the help of a microcontroller the accumulated charge is calculated. When the accumulated charge reaches the target value the drive is stopped.

In other embodiments, the cell may be driven such that gas is always produced, but at varying levels. For example, in some implementations, there may be a latency period between the time at which the cell is energized, or turned on, and the time at which enough gas is generated to dispense contents from the device. This latency period may be decreased by maintaining some measure of gas generation at all times. Specifically, it may be quicker for the cell to produce enough gas to empty the device when the cell is already producing gas, than when the cell is completely de-energized. In some embodiments, to ensure that the substance to be dispensed is not inadvertently released, i.e., by allowing gas to accumulate sufficiently that the flexible membrane is deformed, the rate of gas generation by the cell when the gas is not intended to drive the contents out of the device may be lower than the rate at which gas exhausts or dissipates from the device, e.g., via the dissipation disk.

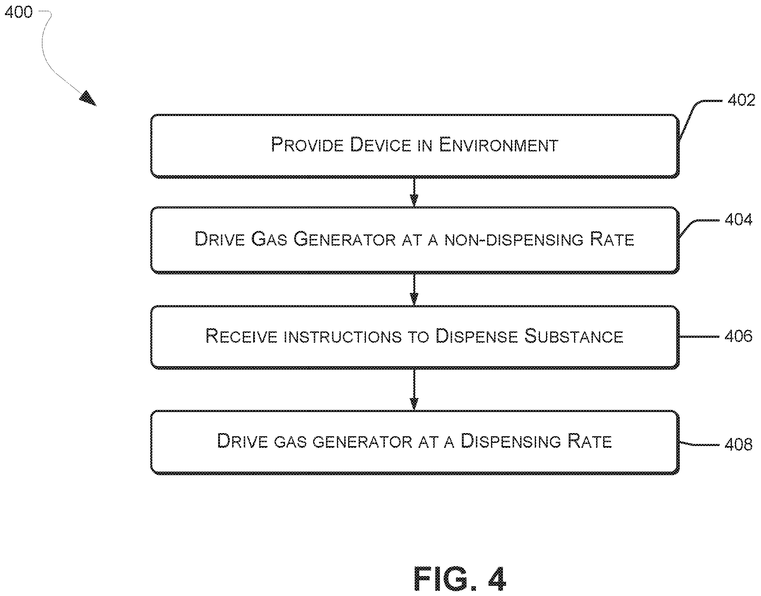

A method 400 of driving the gas generator in the foregoing manner is illustrated in FIG. 4. More specifically, at 402, a device such as those described above is provided in an environment. For example, the device may be in the form of a swallowable capsule and at 402 a patient swallows the capsule. In other implementations, the device may be inserted into a bodily cavity. In these implementations, the device preferably includes a dispensing chamber filled with a substance to be dispensed, a flexible membrane comprising a side of the dispensing chamber, and a gas generator on a side of the membrane opposite the dispensing chamber, i.e., arranged to act on the outside of the dispensing chamber, to compress the dispensing chamber. The gas generator may be contained in an actuator housing, and gas in the actuator housing dissipates from the housing, other than into the dispensing chamber. For example, an exhaust may be provided that allows gas to exit the actuator housing into the surrounding atmosphere.

At 404, the gas generator is driven at a non-dispensing rate. More specifically, the gas generator is driven by controls to produce gas at a non-zero rate that is insufficient to cause the flexible membrane to drive contents from the device. For instance, the non-dispensing rate may be lower than or equal to a rate at which gas dissipates or is otherwise exhausted from the actuator housing. In other embodiments, the gas generation rate may be higher than a rate at which gas dissipates from the device, but not sufficiently higher that gas will accumulate sufficiently to cause dispensing. For example, gas may be allowed to accumulate slowly in the actuator housing, but the rate of accumulation should be such that enough gas will not accumulate between dispenses.

At 406 the device receives an instruction to dispense the substance contained in the dispensing chamber. For example, the device may receive a signal from an external source, e.g., a device controlled by a technician. In other instances, the instruction may come via an on-board source. By way of non-limiting example, the device's controls may include timing controls, such as including a clock, that instructs a timing of dispensing from the device. In another example, the device may include a locational sensor that, upon sensing a position, e.g., in the gastrointestinal tract, instructs the dispensing of the substance. The locational sensor may include any sensor capable of determining a position of the device. For example, the locational sensor may include a pH sensor that determines where the device is in the GI tract. Alternatively, the locational sensor may sense a malady or symptom of a malady, and dispense upon a presence of that malady or symptom.

At 408, the gas generator is driven to dispense the contents of the dispensing chamber. More specifically, gas is generated in an amount and at a sufficient rate that a pressure sufficient to compress the dispensing chamber is achieved.

Accordingly, in the foregoing example, controls may drive the gas generator to produce gas at a lower (but non-zero) rate e.g., at a non-dispensing rate, or at a higher rate, e.g., a dispensing rate. In the normal state, the gas generated by the gas generator is at a sufficiently low rate that it does not deform the flexible membrane, or at least not to a degree that an appreciable amount of the substance will be dispensed from the capsule. As noted above, the non-dispensing rate is a non-zero rate that may be lower than a rate at which gas dissipates from the device. In this manner, the gas generated merely exhausts from the device, without deforming the membrane. The dispensing rate is a rate greater than the rate at which gas dissipates from the device, such that the gas accumulates sufficiently to deform the membrane, and thereby drive the substance from the device.

Although example embodiments have been described in language specific to the structural features and/or methodological acts, the claims are not necessarily limited to the specific features or acts described. Rather, the specific features and acts are disclosed as illustrative forms of implementing the example embodiments.

## Claims

1. A pump (100) comprising:

   an inlet/outlet plate (202) comprising an inlet hole (212) formed through the inlet/outlet plate and an outlet hole (214) formed through the inlet/outlet plate and spaced from the inlet hole; a valve plate (204) adjacent the inlet/outlet plate comprising an inlet valve (216) aligned with the inlet and an outlet valve (218) aligned with the outlet;
   a channel plate (206) arranged adjacent the valve plate (204), on a side of the valve plate opposite the inlet/outlet plate (202), the channel plate comprising a cavity (220) in fluid communication with the inlet and the outlet;
   a flexible membrane (224) proximate the cavity, at least a portion of the flexible membrane and the cavity defining an dispensing volume (118); and
   an actuator (228) arranged to selectively apply a force to the flexible membrane, on a side of the flexible membrane opposite the dispensing volume.

2. The pump (100) of claim 1, wherein the inlet valve (216) is a one-way valve that selectively allows a substance supplied to the inlet to enter the device.

3. The pump (100) of claim 1 or claim 2, wherein the outlet valve (218) is a one-way valve that selectively allows a substance in the device to exit the device into an environment of the device.

4. The pump (100) of any one of claims 1 - 3, further comprising a dissipation plate (208) disposed between the channel plate (206) and the flexible membrane (224), the dissipation plate including one or more apertures (222) defining a portion of the dispensing volume (118).

5. The pump (100) of any one of claims 1 - 4, wherein the actuator (228) comprises a gas generator.

6. The pump (100) of claim 5, further comprising controls configured to control the gas generator to produce gas, selectively, at a non-dispensing rate and at a dispensing rate, wherein the non-dispensing rate is a non-zero rate lower than a rate at which gas dissipates from the pump and the dispensing rate is larger than the rate at which gas dissipates from the pump.

7. The pump (100) of claim 5, further comprising a dissipation film (210) proximate the actuator (228) to allow gas generated by the actuator to dissipate from the device.

8. The pump (100) of claim 7, wherein a rate of dissipation of gas through the dissipation film (210) is less than a rate at which gas is generated by the actuator (228).

9. The pump (100) of claim 8, further comprising a dissipation plate (208) comprising one or more dissipation channels (234), wherein gas that dissipates through the dissipation film exits the device via the dissipation channels.

10. The pump (100) of any one of claims 4 to 9, wherein the dissipation plate (208) is formed on a side of the channel plate (206) opposite the valve plate (204) and the pump (100) further comprises a dissipation film which is disposed on a side of the dissipation plate opposite the channel plate.

11. A fluid delivery device (200), comprising:

a pump (100) according to any one of claims 1 - 10,
wherein the device is configured to deliver a substance/for controlled delivery of some predetermined amount of a substance.

12. The delivery device of claim 11, wherein the delivery device comprises a reservoir (102) containing a substance and the substance is in fluid communication with the inlet hole (212).

13. The delivery device of claim 11 or claim 12, wherein the delivery device is a swallowable delivery device capsule.

**Patentansprüche**

1. Pumpe (100), die Folgendes umfasst:

eine Einlass-/Auslassplatte (202), die ein Einlassloch (212), das durch die Einlass-/Auslassplatte hindurch ausgebildet ist, und ein Auslassloch (214) umfasst, das durch die Einlass-/Auslassplatte hindurch ausgebildet und von dem Einlassloch beabstandet ist; eine zur Einlass-/Auslassplatte benachbarte Ventilplatte (204), die ein Einlassventil (216), das mit dem Einlass fluchtend ausgerichtet ist, und ein Auslassventil (218) umfasst, das mit dem Auslass fluchtend ausgerichtet ist;
eine Kanalplatte (206), die benachbart zur Ventilplatte (204) angeordnet ist, auf einer Seite der Ventilplatte, die der Einlass-/Auslassplatte (202) entgegengesetzt ist, wobei die Kanalplatte einen Hohlraum (220) umfasst, der in Fluidkommunikation mit dem Einlass und dem Auslass steht;
eine flexible Membran (224) in der Nähe des Hohlraums, wobei zumindest ein Abschnitt der flexiblen Membran und der Hohlraum ein Abgabevolumen (118) definieren; und
einen Aktuator (228), der angeordnet ist, um wahlweise eine Kraft auf die flexible Membran auf einer Seite der flexiblen Membran, die dem Abgabevolumen entgegengesetzt ist, auszuüben.

2. Pumpe (100) nach Anspruch 1, wobei das Einlassventil (216) ein Rückschlagventil ist, das es einer dem Einlass zugeführten Substanz wahlweise ermöglicht, in die Vorrichtung einzutreten.

3. Pumpe (100) nach Anspruch 1 oder 2, wobei das Auslassventil (218) ein Rückschlagventil ist, das es einer Substanz in der Vorrichtung wahlweise ermöglicht, aus der Vorrichtung in eine Umgebung der Vorrichtung auszutreten.

4. Pumpe (100) nach einem der Ansprüche 1 bis 3, ferner umfassend eine Ableitungsplatte (208), die zwischen der Kanalplatte (206) und der flexiblen Membran (224) angeordnet ist, wobei die Ableitungsplatte eine oder mehrere Öffnungen (222) umfasst, die einen Teil des Abgabevolumens (118) definieren.

5. Pumpe (100) nach einem der Ansprüche 1 bis 4, wobei der Aktuator (228) einen Gasgenerator umfasst.

6. Pumpe (100) nach Anspruch 5, ferner umfassend Steuerungen, die ausgelegt sind, um den Gasgenerator zu steuern, um Gas zu erzeugen, wahlweise bei einer Nicht-Abgaberate und einer Abgaberate, wobei die Nicht-Abgaberate eine Rate ist, die nicht null und niedriger als eine Rate ist, in der das Gas aus der Pumpe abgeleitet wird, und wobei die Abgaberate höher ist als die Rate, in der das Gas aus der Pumpe abgeleitet wird.

7. Pumpe (100) nach Anspruch 5, ferner umfassend einen Ableitungsfilm (210) in der Nähe des Aktuators (228), um zu ermöglichen, dass durch den Aktuator erzeugtes Gas aus der Vorrichtung abgeleitet wird.

**8.** Pumpe (100) nach Anspruch 7, wobei eine Rate der Ableitung des Gases durch den Ableitungsfilm (210) geringer ist als eine Rate, in der das Gas durch den Aktuator (228) erzeugt wird.

**9.** Pumpe (100) nach Anspruch 8, ferner umfassend eine Ableitungsplatte (208), die einen oder mehrere Ableitungskanäle (234) umfasst, wobei das Gas, das durch den Ableitungsfilm abgeleitet wird, über die Ableitungskanäle aus der Vorrichtung austritt.

**10.** Pumpe (100) nach einem der Ansprüche 4 bis 9, wobei die Ableitungsplatte (208) auf einer der Ventilplatte (204) entgegengesetzten Seite der Kanalplatte (206) ausgebildet ist und die Pumpe (100) ferner einen Ableitungsfilm umfasst, der auf einer der Kanalplatte entgegengesetzten Seite der Ableitungsplatte angeordnet ist.

**11.** Fluidzufuhrvorrichtung (200), umfassend:

eine Pumpe (100) nach einem der Ansprüche 1 bis 10,
wobei die Vorrichtung zur Zufuhr einer Substanz/für die gesteuerte Zufuhr einer gewissen vorbestimmten Menge einer Substanz ausgelegt ist.

**12.** Zufuhrvorrichtung nach Anspruch 11, wobei die Zufuhrvorrichtung einen Behälter (102) umfasst, der eine Substanz enthält, und die Substanz in Fluidkommunikation mit dem Einlassloch (212) steht.

**13.** Zufuhrvorrichtung nach Anspruch 11 oder Anspruch 12, wobei die Zufuhrvorrichtung eine einnehmbare Zufuhrvorrichtungskapsel ist.

## Revendications

**1.** Pompe (100) comprenant :

une plaque d'entrée/sortie (202) comprenant un trou d'entrée (212) formé à travers la plaque d'entrée/sortie et un trou de sortie (214) formé à travers la plaque d'entrée/sortie et espacé du trou d'entrée ; une plaque de clapets (204) adjacente à la plaque d'entrée/sortie comprenant un clapet d'entrée (216) aligné avec l'entrée et un clapet de sortie (218) aligné avec la sortie ;
une plaque de canal (206) agencée adjacente à la plaque de clapets (204), sur un côté de la plaque de clapets opposé à la plaque d'entrée/sortie (202), la plaque de canal comprenant une cavité (220) en communication fluidique avec l'entrée et la sortie ;
une membrane flexible (224) à proximité de la cavité, au moins une partie de la membrane flexible et la cavité définissant un volume de distribution (118) ; et
un actionneur (228) agencé pour appliquer sélectivement une force à la membrane flexible, sur un côté de la membrane flexible opposé au volume de distribution.

**2.** Pompe (100) selon la revendication 1, dans laquelle le clapet d'entrée (216) est un clapet unidirectionnel qui permet sélectivement à une substance fournie à l'entrée d'entrer dans le dispositif.

**3.** Pompe (100) selon la revendication 1 ou la revendication 2, dans laquelle le clapet de sortie (218) est un clapet unidirectionnel qui permet sélectivement à une substance dans le dispositif de sortir du dispositif jusque dans un environnement du dispositif.

**4.** Pompe (100) selon l'une quelconque des revendications 1 à 3, comprenant en outre une plaque de dissipation (208) disposée entre la plaque de canal (206) et la membrane flexible (224), la plaque de dissipation comprenant une ou plusieurs ouvertures (222) définissant une partie du volume de distribution (118).

**5.** Pompe (100) selon l'une quelconque des revendications 1 à 4, dans laquelle l'actionneur (228) comprend un générateur de gaz.

**6.** Pompe (100) selon la revendication 5, comprenant en outre des commandes configurées pour commander le générateur de gaz afin de produire du gaz, sélectivement, à un débit sans distribution et à un débit de distribution, dans laquelle le débit sans distribution est un débit non nul inférieur à un débit auquel du gaz se dissipe à partir de la pompe et le débit de distribution est supérieur au début auquel du gaz se dissipe à partir de la pompe.

**7.** Pompe (100) selon la revendication 5, comprenant en outre un film de dissipation (210) à proximité de l'actionneur (228) pour permettre à du gaz généré par l'actionneur de se dissiper à partir du dispositif.

**8.** Pompe (100) selon la revendication 7, dans laquelle un débit de dissipation de gaz à travers le film de dissipation (210) est inférieur à un débit auquel du gaz est généré par l'actionneur (228).

**9.** Pompe (100) selon la revendication 8, comprenant en outre une plaque de dissipation (208) comprenant un ou plusieurs canaux de dissipation (234), dans laquelle du gaz qui se dissipe à travers le film de dissipation sort du dispositif via les canaux de dissipation.

**10.** Pompe (100) selon l'une quelconque des revendications 4 à 9, dans laquelle la plaque de dissipation (208) est formée sur un côté de la plaque de canal (206) opposé à la plaque de clapets (204) et la pompe (100) comprend en outre un film de dissipation qui est disposé sur un côté de la plaque de dissipation opposé à la plaque de canal.

**11.** Dispositif de distribution de fluide (200), comprenant :

une pompe (100) selon l'une quelconque des revendications 1 à 10,
dans lequel le dispositif est configuré pour administrer une substance/pour une administration contrôlée d'une certaine quantité prédéterminée d'une substance.

**12.** Dispositif d'administration selon la revendication 11, dans lequel le dispositif d'administration comprend un réservoir (102) contenant une substance et la substance est en communication fluidique avec le trou d'entrée (212) .

**13.** Dispositif d'administration selon la revendication 11 ou la revendication 12, dans lequel le dispositif d'administration est une capsule de dispositif d'administration ingérable.

FIG. 1

EP 3 419 695 B1

**FIG. 2**

**FIG. 3**

FIG. 4

**REFERENCES CITED IN THE DESCRIPTION**

**Patent documents cited in the description**

- US 62300542
- US 5049141 A